# EUROPEAN PATENT SPECIFICATION

(11) **EP 1 620 515 B1**
(45) Date of publication and mention of the grant of the patent: **27.12.2006**
(21) Application number: 04731654.2
(22) Date of filing: 07.05.2004
(51) Int. Cl.: C09D 133/00, C09D 167/06, C09D 4/00, C09D 201/00, C07C 69/593, C07C 69/60, C07C 69/40, C07C 69/44, C07C 69/80

(54) **REACTIVE DILUENTS IN COATING FORMULATION**
REAKTIVVERDÜNNER FÜR BESCHICHTUNGSZUSAMMENSETZUNGEN
DILUANTS REACTIFS EN FORMULATION D'ENROBAGE

(30) Priority: 08.05.2003 GB 0310620; 08.05.2003 GB 0310624; 01.04.2004 GB 0407489
(43) Date of publication of application: 01.02.2006
(73) Proprietor: The University of Southern Mississippi Research Foundation, Hattiesburg, MS 39406 (US)
(72) Inventor: GRACEY, Benjamin Patrick, Hull HU12 8AJ (GB); HALLETT, Christopher, Hertfordshire, WD1 1AW (GB)
(74) Representative: Hamer, Christopher K.
(86) International application number: PCT/GB2004/001978
(87) International publication number: WO 2004/099329

(56) References cited:
- WO-A-97/02230
- WO-A-97/02326
- US-A- 5 252 648
- DATABASE CHEMABS [Online] CHEMICAL ABSTRACTS SERVICE, COLUMBUS, OHIO, US; 8 November 1994 (1994-11-08), BADOU, IGNACE ET AL: "Low VOC fast air-drying alkyd coatings . II. Allylic reactive diluents ." XP002290596 retrieved from STN Database accession no. 1995:135829 & POLYMERIC MATERIALS SCIENCE AND ENGINEERING , 70, 334-5 CODEN: PMSEDG; ISSN: 0743-0515, 1993,

## Description

### Background of the Invention

This invention relates to ether esters and acetals of allylic alcohols, a process for producing these ether esters and acetals with low colour, and use of these ether esters and acetals as diluents in paint and coating formulations.

Reactive diluents are usually compounds or mixtures of compounds of relatively low viscosity and relatively high boiling point (or low saturated vapour pressure) which act as solvents during the formulation and processing of paints and coatings. An advantage of reactive diluents is that such diluents are able to copolymerise with components of an alkyd resin. Hence reactive diluents may be used to replace part or all of the traditional solvents normally used in such formulations thereby reducing losses of the solvent to atmosphere on drying of the coating. Use of esters of di- and polyhydric alcohols that have been partially etherified with allyl alcohol as reactive diluents is described in EP-A-0 253 474. However, these esters have relatively high viscosity of around 0.5 poise (50 millipascal seconds (mPa.s)) and therefore can be used only in a limited number of paint formulations. Moreover, allyl alcohol esters also are susceptible to hydrolysis and are therefore capable of releasing undesirable allyl alcohol. In addition, when polymer formulations containing the esters partially etherified with allyl alcohol are subjected to curing using radical conditions, there is a risk of fragmentation of the molecule, which may release undesirable acrolein vapours. During use as solvents, the fragmentation products of higher allylic alcohols, e.g. octadienol, are much less volatile and are therefore less hazardous to persons in proximity to these materials.

WO 97/02230 and WO 97/02326 describe esters of 2,7-octadienol which may be used as reactive diluents in paint and polymer formulations.

In addition, US 5,252,648 describes the use of octadienyl ethers as reactive thinners for coating compositions, knifing fillers and sealing compositions.

Alkyd resins are well-known components of decorative paints (see, for example, "The Technology of Paints, Varnishes and Lacquers" by Martens. C R, Ed., published by Robert Krieger Publishing (1974)) and can be prepared from polybasic acids or anhydrides, polyhydric alcohols and fatty acids or oils. U.S. Patent 3,819,720, describes methods of preparing such alkyd formulations. Alkyd resins are available commercially and are used in coating compositions which usually contain large amounts of solvents (e.g. mineral spirits, aromatic hydrocarbons). Low VOC fast air-drying alkyd coatings. II. Allylic reactive diluents. Badou, I *et al* describe the production of fast air-drying alkyd coatings, and the use of allylic reactive diluents. Other types of paint and coating formulations have been based on fatty acid modified acrylates, unsaturated polyesters and those that have relatively high solids contents.

A known process used to produce ether esters of allylic alcohols is direct alkoxylation of octadienol in the presence of an acidic catalyst followed by reaction of the hydroxy ether so formed with a carboxylic acid under esterification conditions. The use of a strongly acidic catalyst for the alkoxylation of the octadienol and the subsequent esterification results in the formation of products with unacceptable levels of colour. An alternative route is to catalytically telomerise a glycol with butadiene to form an octadienyl glycol ether which is then esterified with a carboxylic acid. These processes are rather complex, give poor overall yields of the ester and form major by-products such as dimerised butadiene and dioctadienyl ethers (also known as glymes). Moreover, the product work-up is far from simple because precious metals such as palladium are used as the telomerisation catalyst and then must be recovered. Also, such processes need a complex distillation process due to the presence of low and high boiling point reaction by-products and the low volatility of the intermediate allylic ethers. Thus, the prior art processes are economically unattractive.

U.S. Patent 4,418,207 describes forming acetylacetoalkyl allyl ethers by reacting allyl alcohols with alkylene oxides followed by reaction with a diketene. Allyloxy derivatives have been described for use as reactive diluents in U.S. Patents 6,130,275 and 6,103,801 and by Zabel et al., Progress in Organic Chemistry 35 (1999) 255-264).

It has now been found that ether esters and acetals of allylic alcohols can be produced in commercially viable yields and purity and are useful as commercially attractive reactive diluents.

### Summary of the Invention

A composition suitable for use as an reactive diluent is prepared by reacting an allylic alcohol with an epoxide in the presence of a suitable catalyst to form a hydroxy ether, which is further reacted with an aldehyde or a carboxylic acid or derivative using a catalyst that does not induce undue polymerization or rearrangement to form an ether ester or acetal composition useful as an reactive diluent.

### Description of the Invention

The present invention is a coating formulation comprising (i) a binder resin and (ii) a reactive diluent comprising an allyloxy compound:

[R*(OCR⁶R⁷-CR⁸R⁹)ₚ-O]ₐ-Z (I)

wherein
- R*: is an allylic hydrocarbyl or an allylic hydrocarbyloxy hydrocarbyl group containing up to 22 carbon atoms;
- R⁶, R⁷, R⁸ and R⁹: represent
(i) the same or different groups selected from H, an alkyl, an alkenyl and an aryl group, or,
(ii) when taken together represent a cyclohexyl group;
- Z: is selected from 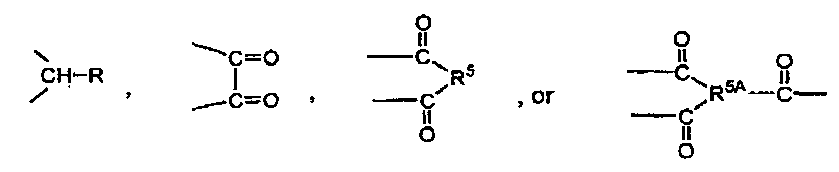 wherein
R⁵ represents a divalent and R^{5A} represents a trivalent saturated or unsaturated hydrocarbylene or oxygenated hydrocarbylene residue derived from a corresponding reactant used for esterification comprising a carboxylic acid, ester, acid halide or anhydride, having 2-20 carbon atoms,
p represents a value from 0 to 5 for each allylhydrocarbyloxy or allyletherhydrocarbyloxy group and is at least 1 for at least one such group, and
a is 2 or 3 and corresponds to the number of bonding sites present in Z.

A suitable process to form the reactive diluent of formula (1) comprises
(a) reacting an allylic alcohol R*OH with an epoxide 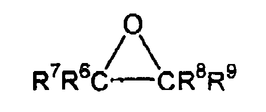 in the presence of a catalyst to form a hydroxy ether;
(b) further reacting the hydroxy ether formed with an aldehyde or a carboxylic acid, acid halide, or anhydride corresponding to the structure of Z, defined above, optionally in the presence of a catalyst incapable of inducing undue polymerisation or rearrangement of the hydroxyether under the reaction conditions; and
(c) recovering the reactive diluent.

In another aspect of this invention, an allylic alcohol described above may be reacted directly with a suitable aldehyde to form a product suitable for use as a reactive diluent, i.e., p may be zero for all groups.

In the process of this invention, one or more suitable allylic alcohols (e.g., Formula II) are contacted with an epoxide in the presence of a suitable catalyst under epoxidation conditions to form a hydroxy ether.

The reactant allylic alcohol, R*OH, used to produce the allylic ether alcohol derivatives of the present invention can be prepared in several ways known to those skilled in the art. For instance, octadienol may be prepared by telomerisation of butadiene and water, which yields a mixture of isomers (predominantly 2,7-octadien-1-ol and a minor amount of 1,7-octadien-3-ol). Alternatively, the reactant allylic alcohol and the saturated analogue R*OH can be produced by the reduction of the corresponding α,β-unsaturated aldehyde (e.g., by hydrogenation), which will generate a mixture of the allylic alcohol and its saturated analogue. Other allylic alcohols may be produced from conjugated dienes via well-known addition reactions. Furthermore, other allylic alcohols may be produced by initially forming an unsaturated ester from an olefin and a carboxylic acid, anhydride, ester or an acid chloride followed by hydrolysis of the ester. This latter reaction may, like some of the other reactions mentioned above, result in a mixture of products which includes *inter alia* the desired allylic alcohol, isomers thereof and saturated analogues thereof. The mixtures of allylic alcohol with the saturated analogue thereof and/or the isomers thereof can be then used as such, or after further purification to isolate the desired allylic alcohol, to prepare the allylic ether alcohol.

In compounds of this invention represented by formula (I) illustrated above, R* has the structure suitably derived from allylic alcohol represented as: 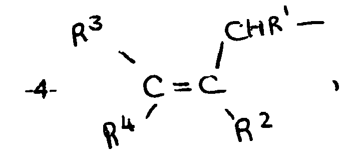 particularly 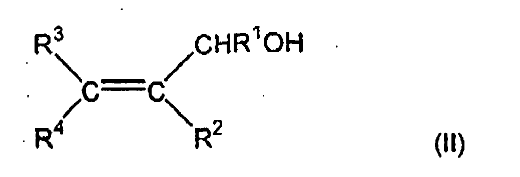 in which formula
- R¹: is H or a C₁-C₄ alkyl group or a hydrocarbyloxy alkyl group containing up to 10 carbon atoms;
- R²: is H or a C₁-C₄ alkyl group or a hydrocarbyloxy alkyl group containing up to 10 carbon atoms;
- R³: is H or a C₁-C₄ alkyl group;
- R⁴: is H, a straight or branched chain alkyl group having up to 8 carbon atoms, an alkenyl group having up to 8 carbon atoms, an aryl group or an aralkyl group having up to 12 carbon atoms, or a hydrocarbyloxy alkyl group having, up to 10 carbon atoms, or,
- R⁴,: when taken together with R¹, forms a cyclic alkylene group, provided R² is H; and
in which formula at least one of the groups R¹, R², R³, and R⁴ is not hydrogen.

R* as used in Formula I may be derived from an allylic alcohol as defined in Formula II. Thus, R* may be a hydrocarbyl group containing an allylic moiety or a similar group further substituted with a hydrocarbyloxy group such as an alkoxy group. Such hydrocarbyl group may contain further unsaturation other than the allylic moiety. Typical examples of R* include 2,7-octadienyl (also known as octa-2,7-dienyl) and 2-ethylhex-2-enyl. R* typically is an allylic-containing hydrocarbyl group containing up to 3 additional unsaturations and containing up to 16 carbon atoms. Preferably, the allylic-containing hydrocarbyl group contains up to 10 carbon atoms and may contain up to two additional unsaturations. R* may also be selected from 2-ethyl-hex-2-en-1; 2,7-octadien-; 2-ethyl allyl; hept-3-en-2-; 4-methyl pent-3-en-2-; 4-t-butoxy but-2-en-1-; 4-(α-methylbenzyloxy)but-2-en-1-; 4-(α-dimethylbenzyloxy)but-2-en-1-; 4-n-butoxy but-2-en-1-; 3-phenyl-2-propen- and 3,5,5-trimethyl-2-cyclohexen-groups.

For the avoidance of doubt, in compositions used this invention described in formula I, the two or three (defined by the quantity, a) allylhydrocarbyleneoxy or allyletherhydrocarbyleneoxy groups, attached to the structure defined as Z, may be the same or different. Thus, there may be one or two allylhydrocarbyleneoxy and one or two allyletherhydrocarbyleneoxy (each having a different value for p) groups containing different substituents represented by R*, R⁶, R⁷, R⁸, and R⁹ in the composition according to formula I. Similarly, the groups derived from an allyl acohol reactant defined as R¹, R², R³, and R⁴ also may be different in each group attached to the structure Z. It is understood that an allyl group may be an allylic hydrocarbyl or a hydrocarbyloxy alkyl group. It is to be further understood that in the definition of "p" the groups referred to by the expression "each allyl hydrocarbyloxy or allyl ether hydrocarbyloxy group" may be depicted by the structure R* (OCR⁶R⁷-CR⁸R⁹)ₚ-O.

In typical allylic alcohol derivatives useful in this invention, R¹, R² and R³ are selected individually from hydrogen, methyl, and ethyl groups. Again, in typical allylic alcohol derivatives useful in this invention, R⁴ is selected from alkenyl groups containing up to 7 carbon atoms preferably containing terminal unsaturation such as a but-3-enyl, pent-4-enyl, and hex-5-enyl groups. Also advantageously R⁴ may be selected from aryl groups or an aralkylene groups having up to 10 carbon atoms, or hydrocarbyloxy alkylene groups having up to 8 carbon atoms. In other typical allylic alcohol derivatives, R⁴ may be alkoxy or alkoxyalkylene containing up to 7 carbon atoms, such as t-butoxy, t-butoxymethylene, iso-propoxy, ethoxy, methoxy, and the like.

Specific examples of allylic alcohols of formula (II) include *inter alia* 2-ethyl-hex-2-en-1-ol (in which R¹ and R³ are H, R² is an ethyl group, and R⁴ is an n-propyl group, and which compound will hereafter be referred to as "2-ethylhexenol" for convenience); 2,7-octadienol; 2-ethyl allyl alcohol; hept-3-en-2-ol; 4-methyl pent-3-en-2-ol; 4-t-butoxy but-2-en-1-ol (also called 1,4-but-2-ene diol mono-tertiary butyl ether (in which R⁴ is a tertiary butoxy methylene group)); 4-(α-methylbenzyloxy) but-2-en-1-ol (also called 1,4-but-2-ene-diol mono α-methylbenzyloxy ether (in which R⁴ is an α-methylbenzyloxy methylene group)); 4-(α-dimethylbenzyloxy) but-2-en-1-ol, (also called 1,4-but-2-ene-diol mono α-dimethylbenzyloxy ether (in which R⁴ is an α-dimethylbenzyloxy methylene group)); 4-n-butoxy but-2-en-1-ol (also called 1,4-but-2-ene diol mono-n-butyl ether (in which R⁴ is a n-butoxy methylene group)); cinnamyl alcohol; and isophorol (in which R² is H, R³ is a methyl group, and R¹ and R⁴ are such that R⁴ represents -CH₂C(CH₃)₂CH₂- and forms a cyclic structure with R¹).

The ethers of allylic alcohols reacted with the carboxylic acids, anhydrides, esters or acid chlorides may themselves be derived either by alkoxylation of the allylic alcohol or, in the case of the ethers of octadienol, by telomerisation. The groups R⁶, R⁷, R⁸ and R⁹ in Formula I typically are derived from an epoxide which is reacted with the allylic alcohol suitably in the presence of a suitable catalyst to form the hydroxy ether.

The epoxidation reaction to form the hydroxy ether can be carried out using one or more of the epoxides which include *inter alia* ethylene oxide, propylene oxide, butadiene mono-oxide, cyclohexene oxide and styrene oxide. The amount of epoxide used for this step would depend upon the number of alkoxy groups desired in the hydroxy ether. The amount of epoxide used is suitably in the range from 0.1 to 20 moles, preferably from 1 to 5 moles based on the allylic alcohol reactant.

The epoxidation step suitably is carried out in the presence of a base catalyst suitable to open the epoxide in the epoxidation reaction. Examples of base catalysts known to the art that may be used include alkali metal hydroxides and alkoxides such as sodium or potassium hydroxide and alkoxide and other metal salts such as potassium acetate. A typical base catalyst is potassium t-butyl butoxide.

The epoxidation reaction is suitably carried out at a temperature in the range from 50 to 180°C, preferably from 60 to 140°C, and typically is conducted in a suitable non-reactive diluent such as a liquid alkane or cycloalkane. The reaction pressure for this step is suitably autogenous and preferably is from 100 to 700 KPa.

The hydroxy ether formed in this step typically is separated from the reaction mixture by use of a suitable neutralisation agent, such as magnesium silicate, then filtered to remove the neutralising agent and the salt of neutralisation so formed to leave behind filtrate comprising the desired hydroxy ether.

The hydroxy ether so produced in the first step can be used either as such without purification, or, optionally, after purification (e.g. by distillation) for the esterification stage.

The ether esters of this invention can be prepared by reacting, an allylic ether alcohol, such as 2-(octadienoxy) ethanol, with a carboxylic acid, an anhydride, an ester or an acid halide in the presence of a suitable catalyst. Suitable catalysts avoid undue polymerisation or rearrangement during esterification and avoid fragmentation of the compound. Rearrangement/fragmentation of ether esters may give rise to colour formation in diluent formulations and, preferably, is avoided. The esterification of the hydroxy ether with a carboxylic acid, anhydride, ester or acid chloride is suitably carried out using a homogeneous or heterogeneous catalyst which may be amphoteric, weakly acidic or weakly basic.

The nature of the catalyst used for the esterification will depend upon the nature of reactants and target products. The same reactants when reacted in the presence of a different catalyst, such as a weakly acidic or amphoteric catalyst, may yield different products as major constituents. The catalyst typically is an amphoteric catalyst. Especially suitable catalysts include dibutyl tin oxide.

The amount of catalyst used in the esterification reaction mixture will not only depend upon the reactants used but also upon the reaction conditions used.

The esterification reaction is suitably carried out under esterification conditions. More specifically, the esterification reaction is suitably carried out at a temperature in the range from 50 to 240°C. The reaction may be carried out at atmospheric, subatmospheric or superatmospheric pressures. The pressures used are suitably at or below atmospheric and may be in the range from 2 to 150 KPa, preferably from 20 KPa to atmospheric pressure.

The esterification reaction is suitably carried out for a duration of at least 2 to 48 hours. The completion of the reaction may be ascertained by GC analysis using a CP-SIL5 50 m capillary column and a flame ionisation detector.

Upon completion of the reaction, the unreacted materials typically are stripped out by steam stripping or by azeotropic distillation (e.g., using an azeotroping agent such as cyclohexane). The catalyst may then be neutralised or removed as appropriate, the solids filtered and the ester in the filtrate recovered.

In the compound of formula (I), suitable hydrocarbylene residues within the description of R⁵, and R^{5A} may be a linear or branched, aliphatic, cycloaliphatic or aromatic, saturated or unsaturated alkylene group, alkenylene group, arylene group, arenylene group, alkarylene group or an aralkylene group. These hydrocarbylene residues suitably have 3-15 carbon atoms, preferably from 4-15 carbon atoms and even more preferably from 6-15 carbon atoms. These residues may be derived from the hydrocarbylene groups attached to the >C=O function of the corresponding carboxylic acid, anhydride, ester or acid halide (e.g. acid chloride) which is reacted with an etherified allylic alcohol. For instance, in the case of phthalic acid or anhydride, the hydrocarbylene residue is an arylene group. Thus, the carboxylic acid or anhydride reactant may be a di-, tri, or a poly-carboxylic acid or the corresponding anhydride. Specific examples of carboxylic acids, anhydrides, esters or acyl halides that may be used in the reaction include *inter alia* oxalic acid, fumaric acid, maleic acid or anhydride, phthalic acid or anhydride and trimellitic acid or anhydride, succinic acid, adipic acid, α- and/or β-hydromuconic acid, malonic acid, mixtures of acids such as the mixed nylonate acids, diethyl maleate and fumaryl chloride. The identity of the structural component X will dictate the nature of the saturated or unsaturated hydrocarbylene group R⁵, or R^{5A}. For example, for a dicarboxylic acid or anhydride R⁵ may be an alkylene or an arylene group.

If R⁵ or R^{5A} is a saturated or unsaturated oxygenated hydrocarbylene residue, the structure of the residue is basically the same as that for the hydrocarbylene residue described above except that the residue may include additional hydroxy or hydroxy alkyleneoxy functions which may be capable of reacting further with acidic functions. Also, if R⁵ or R^{5A} is derived from an unsaturated acid or anhydride such as maleic acid or anhydride, the structure of the residue may contain additional alkyleneoxy functions arising from the addition to the unsaturated acid or anhydride. Thus, for example, if octadienoxy ethanol is reacted with maleic acid or anhydride, the resultant product would include di-(2-octadienoxy ethyl) 2-(2-octadienoxy ethoxy)succinate.

It will be understood by those skilled in the art that during the esterification of the allylic ether alcohols with acids, anhydrides, acid halides or transesterification to form esters of allylic ether alcohols, this reaction may lead to a mixture of products under some reaction conditions. For example, incomplete transesterification could occur. This will be especially true where lower than one equivalent of allylic ether alcohol is used in the reaction.

In respect of the maleates, for instance, the preparative conditions employed will have a strong influence on the type of material obtained from the esterification (or transesterification) reaction, including those used for producing the esters of the allylic ether alcohols. In addition to the degree of esterification (or transesterification) achieved, there is the possibility that the allylic ether alcohol could add to the maleic double bond. Also, it is possible that samples prepared at relatively higher temperatures could isomerise to the fumarate. Furthermore, some allylic ether alcohols (e.g. octadienol) can undergo other reactions leading to oligomers in which several maleic units are linked through the allylic ether alcohol bridging units.

Hence in the Examples in this specification, it will be seen that some samples of maleates of octadienoxy ethanol were of differing viscosity, possibly due to the different degrees of maleate and fumarate present in the respective samples, and also due to the degree of oligomerisation attained by the samples.

The derivatives of allylic ether alcohols of this invention typically have low volatility and relatively low viscosity. Viscosity typically of such derivatives is below 1500 and more typically below 300 milliPascal seconds (3 poise) and preferably below 150 mPa.s, thereby rendering them a suitable reactive diluent for cured paint, coating, and polymer formulations and especially for formulations comprising alkyd resins. For example, a suitable reactive diluent such as the ester of octadienoxy ethanol with maleic anhydride has a viscosity of 40-80 mPa.s.

A typical reactive diluent of this invention has a boiling point above 250°C and more typically above 300 °C. A higher boiling point or a higher vapour pressure typically is indicative of a material with less odour. Thus, an allylic higher molecular weight derivative containing more carbon atoms will reduce odour and reduce volatile organics which may be environmentally detrimental. However, a higher molecular weight material will dilute the reactive sites and typically increase viscosity. Thus, a balance of properties typically is preferred.

Unless otherwise indicated, the expression "alkylene" as used herein and throughout the specification means a divalent hydrocarbyl group such as a -CH₂-(CH₂)*ₜ*-CH₂- group wherein *t* = 0 or an integer, encountered in a compound such as adipic acid. Similarly, an "arylene" group represents a divalent -Ar- group wherein "Ar" is an aromatic nucleus.

Using the process described above, the following ether esters which are representative of the generic class of compounds claimed can be prepared and used as reactive diluents:
Di-(2-(2,7 octadienoxy)ethyl) dihydromuconate
Di-(2-(2,7 octadienoxy)ethyl) fumarate
Di-(2-(2,7 octadienoxy)ethyl) maleate
Di-(2-(2,7 octadienoxy)ethyl) succinate
Di-(2-(2,7 octadienoxy)ethyl) adipate
Di-(2-(2,7 octadienoxy)ethyl) phthalate
Di-(2-ethylhexenoxy)ethyl dihydromuconate
Tri-(2-(2,7 octadienoxy)ethyl) trimellitate
2-(2-Ethylhexenoxy)ethyl maleate
2-(2-Ethylhexenoxy)ethyl fumarate
2-(2-Ethylhexenoxy)ethyl succinate
2-Ethyl hexenyl-(2-ethylhexenoxy)ethyl maleate
2-Ethyl hexenyl-(2-ethylhexenoxy)ethyl fumarate
Di-(1-(octadienoxy)2-propyl) maleate
Tri-(1-(octadienoxy)2-propyl) trimellitate
Di-(1-(2-ethylhexenoxy)2-propyl) maleate
Tri-(1-(2-ethylhexenoxy)2-propyl) trimellitate
Octadienyl-(2-(2,7 octadienoxy) ethyl) maleate
Octadienyl-(2-(2,7 octadienoxy) ethyl) fumarate

Typical ether esters according to the present invention have low volatility and relatively low viscosity, suitably below 1500 mPa.s, thereby rendering them a suitable solvent component for curable paint and varnish formulations.

According to a further embodiment, the present invention is a process for the preparation of compounds of the formula:

(R*O(CR⁶R⁷-CR⁸R⁹O)ₚ]₂.R⁵ (III)

wherein
R*, R⁵-R⁹, and *p* have the same significance as in formula (III) above,
said process comprising reacting at least two moles of the allylic alcohol or a hydroxyalkyl ether derivative thereof with an aldehyde in the presence of a strongly acidic heterogeneous compound as catalyst.

The acetals according to the present invention have low volatility and low viscosity thereby rendering them a suitable solvent component for cured paint and polymer formulations. These acetals are especially suitable as reactive dilutents for coating formulations comprising binder resins, preferably of alkyd resins.

The aldehyde reactant used in the process may be linear or branched, saturated or unsaturated, cyclic, acyclic or alicyclic, or aromatic or an alkylene aromatic group. Examples of aldehydes that may be used in the reactions of the present invention include *inter alia* formaldehyde, acetaldehyde, butyraldehyde, iso-butyraldehyde, 7-octenal and benzaldehyde. Of the unsaturated aldehydes that may be used, it is preferred that the unsaturated linkage is not in conjugation with the aldehydic carbonyl group.

In the reaction mixture, the molar ratio of the aldehyde to the allyl alcohol or a hydroxy alkyl ether derivative thereof is suitably in the range from 0.5 to 5, preferably from 1 to 3.

The strongly acidic heterogeneous catalyst that can be used in the process of the present invention is suitably a zeolite such as the hydrogen form of zeolite Y, an intercalated clay or a sulphonic acid functionalised ion-exchange resin such as e.g. Amberlyst®15A. Where a functionalised resin is used, this can be in gel or in macroreticular form.

The amount of strongly acidic heterogeneous catalyst in the reaction mixture for a batch reaction is suitably from 0.01 to 10% w/w, preferably from 0.1 to 5% w/w based on the total amount of the reaction mixture. This ratio may not be of great significance when the reaction is operated as a continuous process in a flow reactor. In the latter instance, the LHSV of the reactants over the catalyst is suitably in the range from 0.1 to 20, preferably from 1 to 5 bed volumes per hour.

The reaction is suitably carried out under at a temperature below 80°C, preferably in the range from 0 to 70°C. The reaction between the allylic alcohol or the hydroxyalkyl ether derivative thereof and an aldehyde is exothermic and it may be necessary to use cooling influences on the reactor to keep it below the temperature specified above. On the other hand, it may be necessary initially to marginally heat the reaction mixture and then allowing the mixture to cool before it attains the upper limit within the range specified above.

The reaction may be carried out at atmospheric, subatmospheric or superatmospheric pressures. The pressures used are suitably in the range from 100 to 250 KPa, preferably from 100 to 150 KPa.

The reaction is suitably carried out for a duration of 0.1 to 8 hours, preferably from 2 to 4 hours for a batch reaction. When a continuous process is used the LHSV would be within the ranges specified above. The attainment of reaction equilibrium is ascertained by GC analysis.

Upon completion of the reaction, the reaction mixture is rendered free of the catalyst by filtration and any residual acidic impurities neutralised by the use of weak base which should preferably be in resin form. Thereafter, having neutralised all of the acid therein, the mixture is distilled under reduced pressure to strip off any water, unreacted aldehyde and allyl alcohol therefrom. The removal of unreacted allyl alcohol or a hydroxyalkyl ether derivative thereof is facilitated by steam stripping and avoids the need to boil the acetal product which may undergo discoloration if subjected to heating at high temperature for a prolonged period. The acetal formed is sufficiently stable to be distilled off at reduced pressure but may require a high vacuum e.g. <5mm Hg (0.66 KPa) and a temperature >120°C.

The compositions of the present invention are highly suitable for use as reactive diluents.

The relative proportions of the compounds of this invention used as reactive diluents to the alkyd resin in a formulation can be derived from the ranges quoted in published EP-A-0 305 006. In an example in which the reactive diluents of the present invention replaces all of the traditional solvent, the proportion of reactive diluent to alkyd resin is suitably at least 5:95 parts by weight and may extend to 50:50 parts by weight. A preferable proportion of reactive diluent to alkyd resin is up to 25:75, and more preferably is up to 15:85, parts by weight.

The formulations may contain further components such as catalyst, drier, antiskinning agent, pigments and other additives. The formulations also may need to include water scavengers such as molecular sieves or zeolites where the reactive diluent used is susceptible to hydrolysis. Furthermore, where such water scavengers are used it may be necessary to use them in combination with pigment stabilizers. Where a drier is used this may further contribute towards the solvent content of the formulation. The formulation may comprise one or more of alkyd resins, unsaturated polyesters, or fatty acid modified acrylics.

The diluents of the present invention can be used in a range of resin binder systems including alkyds used in conventional high solids and solvent-free decorative paints, where necessary in the presence of a thinner such as white spirit. These diluents also may be used in other resin systems, especially where oxidative drying and double bonds characterise the binder system. Examples of the latter type are unsaturated polyesters, fatty acid modified acrylics. Such systems are known to the art. For effective use with the reactive diluents of this invention, a paint or coating system contains a resin or binding system (such as an alkyd resin) that will react with the reactive diluent to form chemical bonds, typically upon drying (curing). Such reaction may be with reactive sites, such as carbon-carbon double bonds or through an oxidative process. The reaction of the binding system with the reactive diluent inhibits release of volatile materials during a coating drying or curing phase.

For some uses it is preferable that the free alcohol content of the diluent is minimised in order to facilitate drying of the formulation. The present invention is further illustrated, but not limited, with reference to the following Examples.

### EXAMPLES

All manipulations - unless otherwise specified - were carried out under a nitrogen atmosphere. All the allylic alcohols and allylic ethers derivatives were distilled before use in the preparations of the ether esters. The octadienol was obtained from Fluka Chemicals. The dimethyl maleate, diethyl maleate and trimethyl 1,2,4-benzene tricarboxylate used were commercial products supplied by Aldrich Chemical Co.

### Preparation of the Ether Alcohols:

The mixed isomeric compounds, 1-(2,7-octadienoxy)propan-2-ol and 2-(2,7-octadienoxy)propan-1-ol were prepared by the reaction of 2,7-octadienol with propylene oxide and purified by distillation (see examples below). No attempt was made to separate the two isomers of which the secondary alcohol was the major component e.g.

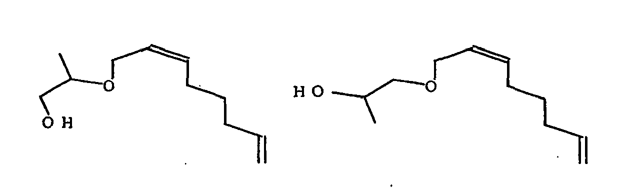

The ethoxylation of the allylic alcohol such as 2,7-octadienol in the presence of a base catalyst such as potassium hydroxide may be carried out in an autoclave which is previously purged with nitrogen, pressured to e.g. about 240 KPa and heated to e.g. about 125°C. The ethylene oxide is then added slowly to the autoclave to maintain a total pressure of e.g. about 520 KPa. The reaction is completed within 4-5 hours. The mixture is then allowed to cool to room temperature and discharged under nitrogen, neutralised with magnesium silicate (Ambrosol®) and filtered to remove neutralised catalyst. A mixed product corresponding to the number of glycol groups added being 1-5 is usually obtained representing yields of around 99%. The mixed product can be analysed for hydroxyl numbers and double bond content to ensure that no unwanted isomerization of the allylic alcohol to a ketone had taken place.

Similarly the 1-(2-ethylhex-2-en-1-oxy)propan-2-ol and 2-(2-ethylhex-2-en-1-oxy)propan-2-ol were prepared by the reaction of 2-ethylhex-2-en-1-ol with propylene oxide. In addition to this, the dipropoxylated derivative of 2-ethylhexenol was prepared by further reaction with propylene oxide. The 2-(2,7-octadienoxy) ethanol (also called octadienoxy glycol ether) and the corresponding octadienoxy diglycol ether feedstocks were prepared by the palladium catalysed telomerisation of butadiene with ethylene glycol and diethylene glycol respectively. It should be noted that, when the octadienoxy ethanol is prepared by telomerisation, the product consists of a mixture of two major isomeric forms, with the linear isomer being predominant. These isomers can be represented as follows:

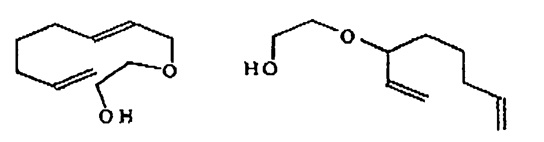

When the octadienoxy ethanol is prepared from the ethoxylation of octadienol, only the linear isomer is obtained.

### Formation of Octadienol Monoglycol Ether:

A three-necked Quickfit® round-bottomed flask was equipped with a thermowell, pressure equalising dropping funnel, condenser, nitrogen top cover, and magnetic follower. To the flask was charged:

| | |
|---|---|
| 150g | 2.7-Octadienol |
| 50ml | Cyclohexane |
| 0.54g | Potassium tertiary butoxide base |

A mixture of the above reactants was heated to 60°C at which temperature a slow reflux of the cyclohexane was observed. Propylene oxide (2 equivalents) was then added to the flask at such a rate that the temperature of reflux was maintained within 5°C of that specified above. The mixture was heated for 8 hours and a GC of the resultant reaction mixture showed approximately 50% conversion to the corresponding monoglycol ether.

The reflux was then maintained for a further 8 hours when conversion increased to 75%. The mixture was then allowed to cool under nitrogen. Acetic acid (5 ml) was then added to quench the base in the reaction mixture. The excess propylene oxide, acetic acid and cyclohexane were removed by rotary evaporation with a water pump. The final mixture was then distilled under vacuum 27 kPa (200 mm Hg) to yield, initially, unreacted octadienol (32g) and finally a mixture of 1-(2,7-octadienoxy)-propan-2-ol and 2-(2,7-octadienoxy)-propan-1-ol (170g). The identity of these compounds was confirmed by ¹H NMR and GC/MS.

### Reaction of Octadienol with Ethylene Oxide:

The following reactants were charged to an autoclave:

| | |
|---|---|
| 300g | 2,7-Octadienol (commercial ex Kuraray, pale yellow) |
| 0.1% | KOH (based on the final product weight) |

The autoclave was purged with nitrogen and pressured to 240 KPa and heated to 125°C. The ethylene oxide (312g) was added slowly to the autoclave to maintain a total pressure of 520 KPa. The reaction was completed within 4.5 hours. The mixture was allowed to cool to room temperature and discharged under nitrogen, neutralised with magnesium silicate (Ambrosol®) and filtered to remove neutralised catalyst. A yield of 605 g (theoretical 612g) represented a yield of 99%. GC analysis showed that a mixed product was obtained corresponding to the number of glycol groups added being 1-5. The mixed product was analysed for hydroxyl numbers and double bond content. This showed that negligible isomerization of the allylic alcohol to a ketone had taken place. For instance, the double bond content found was 7.44 (theoretical 7.75) compared with octadienol content found to be 15.8 (theoretical 15.87). The colour of the mixed product was pale yellow, similar to the starting octadienol.

### Reaction of Octadienol with Ethylene Oxide:

The process described above was repeated except that 2,7-octadienol was distilled prior to use at 130°C, 3mm Hg (to remove the pale yellow colouration). In this example, 509g of the product was recovered (theoretical = 510g). The product obtained was a pale yellow liquid which was lighter than that produced above. Again, the theoretical double bond content (7.751) matched (within experimental error) that obtained from the product (7.61).

### Preparation of Ethoxylated 2-ethyl hexenol:

A 2-litre, stainless steel-lined autoclave was equipped with an oil heating jacket and an overhead stirrer. Ethylene oxide (EO) was supplied through a welded, high integrity stainless steel line, *via* a weigh bomb next to the reactor. The 2-ethyl hexenol was stored over molecular sieves to dry it, and under a nitrogen blanket, when practical, to prevent oxidation. This stored 2-ethyl hexenol was charged to the autoclave. The catalyst used was potassium acetate (KOAc), which was dissolved in the alcohol before charging the reactor. The reaction temperature was generally set at 140-145°C, the catalyst concentration used was between 500-1000 ppm (0.05-0.1 wt.%), and the reaction pressure was ca. 480 KPa (4.8 barg) at 145°C (made up by a nitrogen partial pressure of ca. 280 KPa (2.8 barg) and an EO partial pressure of 200 KPa (2 barg)). The reactor was stirred at 300 rpm and heated to the reaction temperature. The required amount of EO was fed into the reactor by the distributive control system so as to maintain a constant pressure above the reactants. On completion of EO addition, the stirring was continued and the mixture heated for a further half-hour. The reactor was then cooled and purged with nitrogen to remove unreacted EO. The cold product was then discharged and sparged with nitrogen to remove any remaining EO before being filtered through Fluorosil® magnesium silicate.

The final product was analysed by GC. The GC used was a CP-SIL5 capillary column, 50 m long, starting at 50°C, then ramped to 250°C at 10°C/minute, and holding at 250°C for 15 minutes.

GC results (based on peak area %) in which "n" represents the number of glycol units in the product are shown in Table 1 below:

**TABLE 1**

| n = 0 (wt %) | n =1 (wt %) | Conversion of n=0 | Selectivity to n=1 | Yield of n = 1 |
|---|---|---|---|---|
| 41.95 | 25.15 | 58.05 | 43.32 | 25.15 |

The cold crude ethoxylate mixture was then discharged from the autoclave and sparged with nitrogen to remove any traces of the unreacted ethylene oxide. The mixture was then filtered filtered through magnesium silicate to remove the KOAc catalyst. The crude product was then distilled under reduced pressure (typically 50 mbar, 65°C) to remove the unreacted 2-ethyl hexenol. The temperature was then raised to about 93°C to separate pure monoethoxylated 2-ethyl hexenol from the other polyethoxylated product. The identity of the product was confirmed by GC and ¹H and ¹³C NMR analysis.

### Preparation of Esters of Ether Alcohols:

The following apparatus was assembled:
A three-necked Pyrex Quickfit® round-bottomed flask was equipped with two side arms, a magnetic follower and a heater stirrer mantle. The top of each of the three necks of the flask was connected respectively to a packed column, a liquid heads take-off assembly and a controllable source of vacuum or nitrogen top cover.

The temperature of the flask contents was controlled by means of a thermocouple inserted into one of the flask side arms. The remaining side arm, when not stoppered, was used for purging the apparatus with nitrogen prior to use and for charging the reactants. The apparatus was purged with nitrogen to displace any air and moisture, and then the allylic ether alcohol was added to the flask. The allylic ether alcohol was purged of any oxygen by means of a nitrogen sparge. Once degassed, the trimethyl 1,2,4-benzenetricarboxylate was added. 1.05 Equivalents of allylic alcohol were used per carboxylate functionality in the tricarboxylate. On the weight of reactants in the flask, a transesterification/esterification catalyst (e.g. dibutyl tin oxide) was added at typically 0.1-1% w/w.

The mixture was heated to 160°C under a nitrogen atmosphere during which displaced low boiling point alcohol was collected in the heads take-off. This distillation was continued until heads material ceased to be collected. The reaction pressure was then lowered to 50mm Hg and held for 6 hrs to complete the reaction. The applied vacuum was then increased to 1 mbar to distill across any unreacted alcohol together with a minor by-product acetate ester of the alcohol. It was found that use of a vacuum for the reaction was beneficial as it reduced the amount of esterification by rapid disengagement of the acetic acid by-product.

The progress of the reaction and distillation was monitored by gas chromatography. A target of less than 2% free alcohol was set in the kettle product. Once this had been achieved the reaction mixture was allowed to cool to room temperature and filtered through a short bed of chromatography grade silica to remove any traces of the dibutyl tin oxide catalyst. The identity of the product was confirmed by GC and ¹H, and ¹³C NMR analysis as follows:
The progress of the reaction and distillation was monitored by gas chromatography. A Hewlett-Packard hp 5890 machine fitted with an auto-sampler and a flame ionisation detector was used for this purpose. The chromatographic column used was a commercially available 50 metre CPSIL5 packed column. Typically, the GC oven was preheated to 50°C with the temperature being ramped at 5°C per minute until it reached 270°C where it was maintained isothermally for 2 hours. The injection port was kept at a constant temperature of 260°C. The run lasted approximately 165 minutes for each sample.

The GCMS data was obtained on a Hewlett-Packard 5890 gas chromatograph linked to a VG Trio-1000 mass spectrometer. The analyse was a quadrupole system and the ionisation source generated an electron beam which caused ionisation of the samples by electron impact. The source used was typically:

| | |
|---|---|
| lonisation Energy = | 70 eV |
| lonisastion Current = | 230 µA |
| Ion Acceleration = | 2.0V |
| Pressure = | 10⁻⁴ to 10⁻⁵ Bar |
| Temperature = | 200°C |
| Calibrant = | Heptacoas (Perfluoro tri-n-butylamine) |

A target of less than 5% free alcohol was set in the kettle product. Once this had been achieved the reaction mixture was allowed to cool to room temperature and filtered through a short bed of chromatography grade silica to remove the dibutyltin oxide catalyst. The identity of the product was confirmed by GC, ¹H and ³C NMR analysis.

Compounds of the (2-(octadienoxy)ethyl) trimellitate were prepared by this method.

### Reaction of an allylic ether-alcohol with a maleate

The following apparatus was assembled:
A three-necked Pyrex Quickfit® round-bottomed flask was equipped with two side arms, a magnetic follower and a heater stirrer mantle. The top of each of the three necks of the flask was connected respectively to a packed column, a liquid heads take-off assembly and a controllable source of vacuum or nitrogen top cover.

The temperature of the flask contents was controlled by means of a thermocouple inserted into one of the flask side arms. The remaining side arm, when not stoppered, was used for purging the apparatus with nitrogen prior to use and for charging the reactants. The apparatus was purged with nitrogen to displace any air and moisture, and then the allylic ether alcohol was added to the flask. The allylic ether alcohol was purged of any oxygen by means of a nitrogen sparge. Once degassed, the dialkylmaleate (dimethyl maleate, diethyl maleate) was added. On the weight of reactants in the flask, a transesferication/esterification catalyst (e.g. dibutyl tin oxide) was added at typically 0.1-1% w/w.

The mixture was heated to 160°C under a nitrogen atmosphere during which displaced low boiling point alcohol was collected in the heads take-off. This distillation was continued until heads material ceased to be collected. The reaction pressure was then lowered to 50mm Hg and held for 6 hrs to complete the reaction. The applied vacuum was then increase to 1m bar to distill across any unreacted alcohol.

Ester products containing compounds were prepared by this method, such as:
di-((2-octadienoxy)ethyl) maleate,
2-(2-ethylhexenoxy)ethyl maleate, and
2-(2-ethylhexenoxy)ethyl fumarate.

### Reaction of an acid chloride with an allylic alcohol to form an ester:

The following apparatus was assembled:
A three-necked Pyrex Quickfit® round-bottomed flask was equipped with two side arms, a magnetic follower and a heater stirrer mantle. The top of each of the three necks of the flask was connected respectively to a pressure equalising dropping funnel, a double condenser and a controllable source of vacuum or nitrogen top cover.

The apparatus was purged with nitrogen to displace any air and moisture, then the allylic alcohol and dry cyclohexane were added to the flask. The acid chloride was then loaded into the dropping funnel. These reactants were purged of any oxygen by means of a nitrogen sparge. Once degassed, the apparatus was evacuated by connecting it to a water pump. Allylic alcohol (0.9 equivalents) was used per carboxylic acid moiety in the reactant acid chloride.

The mixture was brought to reflux by heating to approximately 50°C under vacuum and the acid chloride was added dropwise over the period of an hour. The reactants were kept under reflux for 2 hours after the acid chloride addition had been completed. The reaction mixture was then distilled on a rotary evaporator to remove the cyclohexane, unreacted acid chloride and allylic alcohol.

The progress of the reaction and distillation was monitored by gas chromatography. A target of less than 5% free alcohol was set in the kettle product. Once this had been attained, the identity of the product was confirmed by GC, ¹H and ¹³C NMR analysis.

### Identification of Di-(2-ethyl hexeneoxy) ethanoxy succinate:

The GC analysis of the purified product revealed a series of three peaks of different intensity with retention times in the range of 90-98 minutes. These peaks were attributable to *cis*/*trans*-isomerisation of the double bond in the allylic alcohol.

The level of free alcohol in these samples was determined by GC calibration to be below 2% by weight. The GCMS spectra also showed a similar pattern with three distinct peaks of varying intensities at retention times of between 63 and 71 minutes. A small peak was also noticeable with a retention time of ca. 30 minutes which probably corresponds to the (2-ethyl hexenoxy) ethyl succinate monoester. No parent ion was observed in the GCMS fragmentation pattern. The rest of the fragmentation pattern was indistinguishable to that obtained when the free alcohol was analysed.

### Identification of Di-(2,7-octadienoxy) ethanoxy succinate:

The GC analysis of the purified product revealed a series of three peaks of different intensity with retention times in the range of 74-90 minutes. These peaks were attributable to *cis*/*trans*-isomerisation of the double bond in the allylic alcohol.

A small peak was also observed in the 65-70 minutes range which was attributable to 1,7-octadienoxy ethanol isomer which was present at approximately 10% levels in the allylic alcohol feedstock. The level of free alcohol in these samples was determined by GC calibration to be below 1% by weight. The GCMS spectra also showed a similar pattern with three distinct peaks of varying intensities at retention times of between 83 to 90 minutes. A small peak was also noticeable with a retention time of ca. 32 minutes which probably corresponds to the (2,7-octadienoxy)ethyl succinate monoester. No parent ion was observed in the GCMS fragmentation pattern. A small peak was observed at 65 minutes which corresponded to the isomeric form of the di-ester product. The rest of the fragmentation pattern was indistinguishable to that obtained when the free alcohol was analysed.

### Preparation of Di-(2,7-Octadienyl) Acetaldehyde Acetal

To a three-necked round-bottomed quick-fit flask equipped with a magnetic follower, reflux condenser and a nitrogen top cover was charged:
6 g Amberlyst® 15H (wet weight prior to swelling in acetone 1 hr, filtration and washing with further acetone and air drying)
127.06g 2,7-Octadienol
70g Acetaldehyde

The reaction was started by stirring the mixture. An exotherm occurred causing the reacted acetaldehyde to reflux. The stirring was continued for 4 hrs and the liquid phase analysed by GC. The assignment of peaks in the product was as follows: 3.02 min - acetaldehyde; 10.36 - acetone from resin; 20.55 - 2,7-octadienol; 43.59 and 44.20 - acetal; and 50 min - unknown materials not present in the feedstocks. By area %, the approximate conversion of octadienol was 55%.

The mixture was worked up by removal of the Amberlyst® catalyst by filtration, and subsequent rotary evaporation of the mixture using a rotary pump served to remove the unreacted acetaldehyde, acetone and octadienol. A GC analysis of the pale yellow odourless product of this treatment showed that the product consisted predominantly of one high boiling point compound with two major isomers (<5% area of unreacted octadienol and high boilers). Proton (¹H) nmr confirmed that the product was the acetal of acetaldehyde. The presence of more than one isomer was confirmed to be due to cis/trans- isomerization of the allylic bond. The reaction selectivity was >95%, 75 g of pale yellow product was obtained.

### Preparation of Di-(2,7-Octadienyl) Aldehyde Acetals of N-and Iso-Butyraldehyde

Two experiments were conducted in parallel using iso-butyraldehyde and n-butyraldehyde:
To a three-necked round-bottomed flask equipped with a magnetic follower, reflux condenser and nitrogen top cover was charged:
   5g Amberlyst® 15H (wet weight prior to swelling in acetone 1 hr, filtration and washing with further acetone and air drying)
   450 ml 2,7-Octadienol
   350 ml Aldehyde

The reaction was started by stirring the mixture. An exotherm occurred causing the mixture to heat up by 10°C. The stirring was continued for 8 hrs.

The mixture was worked up by removal of the Amberlyst® catalyst by filtration, and then any residual acid was removed by treatment with 5g of Amberlyst® A21 for 1 hr. After filtering the mixture to remove this resin, the mixture was rotary evaporated (a rotary pump served to remove the majority of the unreacted aldehyde and octadienol).

Unreacted octadienol was removed as follows:
(a) A round-bottomed flask was charged with the crude acetal and an equal volume of water (made slightly basic by addition of 0.1% NaHCO₃). On top of this was placed an upper phase azeotrope removal apparatus and a condenser and nitrogen top cover was provided. The contents of the flask were heated to reflux and unreacted octadienol recovered overhead. Cooling of the flask left a two-layer mixture the top layer of which was the purified acetal. Yield of the acetal based on the aldehyde was 40-50% by weight, selectivity as determined by GC analysis was >97%. The product obtained was pale straw yellow in colour and contained 0.6% w/w water as determined by Karl Fischer analysis.
(b) A round-bottomed flask was charged with the crude acetal and equipped with a short length of packed column and a still head/receiver apparatus. The pressure of the system was reduced to 1-2KPa, the flask was heated to 120°C and water injected by means of a syringe needle (5 ml/min). The unreacted octadienol was recovered from the heads in a dry state and the purified acetal was obtained from the flask in a dry state (0.05% w/w water). Yield and selectivity were identical (within experimental error) with that described in method (a) above.

### Preparation of the 2,7-Octadienol Acetal using Formaldehyde

The following apparatus consisting of a heater stirrer mantle controlled by a eurotherm controller, a three-necked round-bottomed flask, stirrer bar, condenser and nitrogen top cover was assembled. The flask was charged with:
14.84g Paraformaldehyde
70g 2,7-Octadienol
5g Amberlyst® 15H resin
100 ml 1,2-Dimethoxyethane

The above mixture was heated to 70°C (the temperature at which paraformaldehyde starts to crack) with stirring. Slow deposition of paraformaldehyde was noted which limited the rate of formation of the corresponding acetal. After 5hrs, the mixture was sampled by and analysed by GC. This demonstrated that about 50% conversion to the acetal had been selectively achieved.

The mixture was then filtered using filter aid to remove the resin and any uncracked paraformaldehyde from the resultant yellow solution. This solution was stirred with 5g Amberlyst® A21 for one hour to remove any acidic impurities.

The mixture was rotary evaporated to remove the ether and then steam stripped under reduced pressure (1-2 KPa) to recover the octadienol and remove any formldehyde for re-use. The product from this step had a purity of >95% as determined by GC analysis but had a bright yellow colour. It was found that this colour could be minimised by preparing a solution of formaldehyde in octadienol prior to adding the Amberlyst ®15H resin catalyst. The formaldehyde solution could be conveniently prepared either by dissolving gaseous formaldehyde in the octadienol or by 1/1 extraction of formalin with octadienol followed by vacuum stripping of water. In the latter case when the reaction was repeated with Amberlyst®15H resin, the co-solvent was not used and the reaction proceeded rapidly to give about 60% conversion based on the formaldehyde reactant as determined by GC analysis. The product work up was conducted as previously except that after neutralisation of the catalyst, the steam stripping was conducted first at 100°C at 50 KPa pressure to recover unreacted formaldehyde as a formalin solution and then at 120°C at 1 KPa to recover octadienol. The resultant product was a pale yellow liquid with a yield of 60% and a selectivity of >95% based on octadienol.

The physical properties of the various acetals produced can be summarised as follows:

| Aldehyde in Acetal | Viscosity (mPa.s) | Boiling Point (°C) |
|---|---|---|
| Formaldehyde | 45.6 | 324 |
| Acetaldehyde | 46 | 327 |
| N-butyraldehyde | 55.8 | 342 |
| lso-butyraldehyde | 55.3 | 330 |

### Acetal of 2-ethyl hexenal

The process of preparation of di-(2,7-octadienyl) acetaldehyde acetal described above was repeated but this time using 2-ethyl hexenal instead of acetaldehyde as the aldehyde reactant. The resultant product was an acetal of 2-ethyl hexenal.

### Testing of reactive diluents in paint formulations:

A good reactive diluent must meet a range of criteria including low odour and toxicity, low viscosity and the ability to "cut" the viscosity of the paint to facilitate application on the surface to be coated therewith. Furthermore, the diluent should not have a markedly adverse effect on the properties of the paint film such as drying speed, hardness, degree of wrinkling, durability and tendency to yellowing. The reactive diluents described above have therefore been tested in paint applications using both clear and pigmented paints. The diluents have been compared with paints formulated using white spirit, a conventional thinner.

### Unpigmented "Clearcoat" Formulations:

Unpigmented ("clearcoat") paint formulations were prepared using a high solids alkyd resin SETAL® EPL 91/1/14 (ex AKZO NOBEL, and described in "Polymers Paint and Colour Journal, 1992, 182, pp. 372). In addition to the diluent, Siccatol® 938 drier (ex AKZO NOBEL) and methyl ethyl ketone-oxime (hereafter "MEK-oxime") anti-skinning agent were used. Where used, the white spirit was Exxon type 100. The nominal proportions of the above materials in the paint formulations were:

**TABLE 2**

| Materials | Parts by weight |
|---|---|
| Resin + Diluent | 100.0 |
| Siccatol 938 | 6.7 |
| MEK-oxime | 0.5 |

Note that for white spirit formulations only, the proportions of drier and antiskinning agent were calculated on the basis of the resin only. Thus, the concentration of these components in the paint was lower than for other diluents.

### Preparation of Clearcoat Formulations:

Alkyd resin and diluent were mixed in glass jars for 2 hours (e.g. using a Luckham multi-mix roller bed) in the proportions required to achieve a viscosity (measured via the ICI cone and plate method using a viscometer supplied by Research Equipment (London) Limited) of 6.8 ± 0.3 poise (680 ± 30 mPa.s). Typically, this resulted in a mixture which was ca. 85% w/w resin. If further additions of diluent or resin were required to adjust the viscosity to 6.8 ± 0.3 poise (680 ± 30 mPa.s), a further hour of mixing was allowed. The required quantity of drier was added and, after mixing (1 hour), the required amount of anti-skinning agent was added. After final mixing for at least 30 minutes, the viscosity of the mixture was measured to ensure that the viscosity was between 6.1 and 6.9 poise (610 and 690 mPa.s).

The mixture ("formulation") was then divided into two jars so as to leave ca. 10-15% v/v headspace of air in the sealed jars. One of the jars was stored at 23°C. in darkness for 7 days before paint applications tests were performed. The second jar was stored ("aged") at 35°C in daylight for 14 days before applications tests were performed.

### Tests for Clearcoat Formulations:

### Application of paint film:

Thin films were applied to cleaned glass test plates using Sheen cube or drawbar applicators with a nominal 75µm gap width.

### Viscosity:

The viscosity of each formulation was measured according to BS 3900 Part A7 with an ICI cone and plate viscometer (supplied by Research Equipment (London) Limited) at 23°C and at a shear rate of 10,000 reciprocal seconds.

The viscosity cutting power ("let-down" or "dilution" effect) of each diluent was measured with the above instrument and using mixtures of alkyd and diluent with a range of compositions. "Let-down" curves were plotted as % Solids (resin) versus Viscosity (poise). The viscosity of each diluent was measured at 23°C using a suspended level viscometer. Densities of the diluents were taken as an average of three readings made at 23°C using density bottles with a nominal 10 cm³ capacity, calibrated with water.

### Drying Performance:

Drying performance was measured using films applied to 30 cm x 2.5 cm glass strips and BK drying recorders. The BK recorders were enclosed in a Fisons controlled temperature and humidity cabinet so that the drying experiment could be performed at 10°C and at 70% relative humidity. Sample performance was assessed on the basis of the second stage of drying (dust drying time, T2).

### Pencil Hardness:

Films applied to 20 cm x 10 cm glass plates were dried for 7 days on the laboratory bench at 23°C and 55% relative humidity. The pencil hardness of each sample was measure using the method described in ASTM No. D3363-74. Each plate was then heated at 50°C (4 days) and the pencil hardness measurement was repeated.

### Incorporation of the Diluent into the Paint Film:

For some of the reactive diluents described below, further evidence of the degree of incorporation of the reactive diluent into the paint film was obtained. A good "reactive" diluent should, rather than evaporating, form chemical bonds with the resin and become bound into the polymer network of the dried paint film. The amount of diluent which evaporated during drying, and the amount of diluent which could be extracted from the cured paint film, and therefore was not bound into the polymer network, was determined.

Those skilled in the art know that day-to-day fluctuations in conditions can introduce some variability into experimental data. To minimise these errors, the tests presented below were conducted as follows: five to eight paint formulations were prepared simultaneously and comprised one reference (white spirit) and four to seven reactive diluent-based paints. These samples were tested at the same time under identical conditions. Comparison of performance data from within these groups of formulations allowed errors due to random sources to be minimised. Hence in the following examples the reader will realise that the apparent variation in performance data from some diluents results from the use of different paint formulations made on different days from the same diluent.

### Tests of Reactive Diluents in Pigmented Paint Formulations:

The following Examples demonstrate that the molecules described above are suitable for use as reactive diluents in paint formulations.

### Incorporation of Diluent into Paint Film:

The data in Table 3 below illustrate the greater degree to which reactive diluents based on esters of allylic ether alcohols have incorporated into the paint film (after 24 hours of drying), in comparison with esters of an allylic alcohol. The ester tested is a maleate. This is a considerable advantage since unincorporated compounds can have adverse effects, eg plasticization of the film, and increased tackiness.

**TABLE 3**

| Solvent | Extractable Solvent | Volatile Solvent | Incorporated Solvent |
|---|---|---|---|
| Di-(2-(2,7-octadienoxy)ethyl) maleate | 0.05 | 0.00 | 99.95 |

### Viscosity:

Table 4 shows that the compounds of the present invention have relatively low viscosity and are suitable for use as reactive diluents.

**TABLE 4**

| Sample | Viscosity (mPa.s) | Density (g/l) |
|---|---|---|
| | At 23°C | At 23°C |
| Di-(2-(2,7 octadienoxy) ethyl) maleate | 82.79 | 1.0303 |
| Tri-(2-(2,7 octadienoxy) ethyl) trimellitate | 299.00 | 1.0740 |
| Tri-(1-(2,7 octadienoxy) propan-2-yl) mellitate | 149.00 | 1.0020 |

### Drying Speed & Hardness:

The results in Tables 5-7 show that paints based on the reactive diluents of the present invention reach the dust dry stage of drying within about 3 hours of a traditional white spirit-based paint, and have similar pencil hardness. These properties are regarded as satisfactory by the industry. Furthermore, it is notable from the results in these Tables that the use of esters of allylic ether alcohols does not affect the film properties adversely when compared with the allylic alcohol esters.

**TABLE 5**

| Solvent | Drying times (hours) | |
|---|---|---|
| | Fresh | Aged |
| Di-(2-(2,7 octadienoxy) ethyl) maleate | 7.2 | 7.0 |
| White Spirit* | 3.7 | 3.4 |

| | | |
|---|---|---|
| *Comparative tests - not according to this invention | | |

**TABLE 6**

| Solvent | Drying Times (Hours) | |
|---|---|---|
| | Fresh | Aged |
| Tri-(2-(2,7 octadienoxy) ethyl) trimellitate | 6.1 | 7.1 |
| Tri-(1-(2,7 octadienoxy) propan-2-yl) trimellitate | 6.7 | 7.2 |
| White Spirit* | 3.4 | 3.8 |

| | | |
|---|---|---|
| *Comparative tests - not according to this invention | | |

**TABLE 7**

| Solvent | Pencil hardness measurements | | | |
|---|---|---|---|---|
| | Initial Pencil | Initial Scratch | Final Pencil | Final Scratch |
| Di-(2-octadienoxy ethyl) maleate | 5B | 4B | B | HB |
| 2-octadienoxy di-octadienyl succinate* | 6B | 5B | 4B | 3B |
| 2-(2-octadienoxy ethoxy) di-(2-octadienoxy ethyl) succinate | 4B | 3B | 3B | 2B |
| White Spirit* | 3B | 2B | B | HB |

| | | | | |
|---|---|---|---|---|
| *Comparative tests - not according to this invention | | | | |

## Claims

1. A coating formulation comprising (i) a binder resin and (ii) a reactive diluent comprising an allyloxy compound:
[R*(OCR⁶R⁷-CR⁸R⁹)ₚ-O]ₐ-Z (I)
wherein
R* is an allylic hydrocarbyl or an allylic hydrocarbyloxy hydrocarbyl group containing up to 22 carbon atoms;
R⁶, R⁷, R⁸ and R⁹ represent
(i) the same or different groups selected from H, an alkyl, an alkenyl and an aryl group, or,
(ii) when taken together represent a cyclohexyl group;
Z is selected from 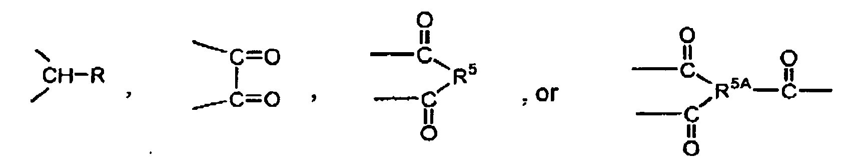 wherein
R⁵ represents a divalent and R^{5A} represents a trivalent saturated or unsaturated hydrocarbylene or oxygenated hydrocarbylene residue derived from a corresponding reactant used for esterification comprising a carboxylic acid, ester, acid halide or anhydride, having 2-20 carbon atoms,
p independently represents a value from 0 to 5 for each allylhydrocarbyloxy or allyletherhydrocarbyloxy group and is at least 1 for at least one such group, and
a is 2 or 3 and corresponds to the number of bonding sites present in Z.

2. A coating formulation of claim 1 wherein the binder resin comprises an alkyd resin.

3. A coating formulation of claim 1 wherein R* has the structure: 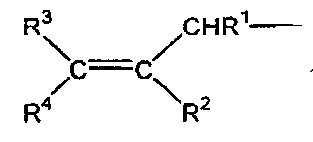 wherein
R¹ is H or a C₁-C₄ alkyl group or a hydrocarbyloxy alkyl group containing up to 10 carbon atoms;
R² is H or a C₁-C₄ alkyl group or a hydrocarbyloxy alkyl group containing up to 10 carbon atoms;
R³ is H or a C₁-C₄ alkyl group;
R⁴ is H, a straight or branched chain alkyl group having up to 8 carbon atoms,
an alkenyl group having up to 8 carbon atoms,
an aryl group or an aralkyl group having up to 12 carbon atoms, or
a hydrocarbyloxy alkyl group having up to 10 carbon atoms, or,
R⁴, when taken together with R¹, forms a cyclic alkylene group, provided R² is H; and
in which at least one group, R¹, R², R³, or R⁴, is not hydrogen.

4. A coating formulation of claim 1 wherein R* is derived from allylic alcohols of the formula: 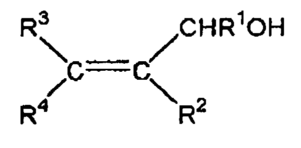 wherein
R¹ is H or a C₁-C₄ alkyl group or a hydrocarbyloxy alkyl group containing up to 10 carbon atoms;
R² is H or a C₁-C₄ alkyl group or a hydrocarbyloxy alkyl group containing up to 10 carbon atoms;
R³ is H or a C₁-C₄ alkyl group;
R⁴ is H, a straight or branched chain alkyl group having up to 8 carbon atoms,
an alkenyl group having up to 8 carbon atoms,
an aryl group or an aralkyl group having up to 12 carbon atoms, or
a hydrocarbyloxy alkyl group having up to 10 carbon atoms, or,
R⁴, when taken together with R¹, forms a cyclic alkylene group, provided R² is H; and
in which at least one group, R¹, R², R³, or R⁴, is not hydrogen.

5. A coating formulation of Claim 3 or 4 wherein R¹, R² and R³ are selected individually from hydrogen, methyl, and ethyl groups and R⁴ is selected from alkenyl groups containing up to 7 carbon atoms, aryl group or an aralkylene groups having up to 10 carbon atoms, or hydrocarbyloxy alkylene groups having up to 8 carbon atoms.

6. A coating formulation of Claim 5 wherein R* is selected from 2-ethyl-hex-2-en-1-; 2,7-octadien-; 2-ethyl allyl; hept-3-en-2-; 4-methyl pent-3-en-2-; 4-t-butoxy but-2-en-1-; 4-(α-methylbenzyloxy)but-2-en-1-; 4-(α-dimethylbenzyloxy)but-2-en-1-; 4-n-butoxy but-2-en-1-; 3-phenyl-2-propen- and 3,5,5-trimethyl-2-cyclohexen-groups.

7. A coating formulation of Claim 2 or 3 wherein the allylic alcohol is selected from the group consisting of 2-ethyl-hex-2-en-1-ol; 2,7-octadienol-; 2-ethyl allyl alcohol; hept-3-en-2-ol; 4-methyl pent-3-en-2-ol; 4-t-butoxy but-2-en-1-ol; 4-(α-methylbenzyloxy)but-2-en-1-ol; 4-(α-dimethylbenzyloxy)but-2-en-1-ol; 4-n-butoxy but-2-en-1-ol; cinnamyl alcohol; and isophorol.

8. A coating formulation of any preceding claim wherein each hydrocarbylene residue R⁵ and R^{5A} is a linear or branched, aliphatic, cycloaliphatic or aromatic, saturated or unsaturated alkylene group, alkenylene group, arylene group, arenylene group, alkarylene group or an aralkylene group containing up to 15 carbon atoms.

9. A coating formulation of any of Claims 1 to 8 comprising one or more of alkyd resins, unsaturated polyesters, or fatty acid modified acrylics.

10. A coating formulation of Claim 9 wherein the relative proportions of the reactive diluent to alkyd resin is in the range from 5:95 to 50:50 parts by weight.

11. A coating formulation of any of Claims 1 to 10 containing one or more further components selected from the group consisting of a catalyst, a drier, antiskinning agent, pigments, water scavengers, and pigment stabilizers.

12. A process to form a reactive diluent of formula (I) defined in Claim 1 comprising:
(a) reacting an allylic alcohol R*OM with an epoxide 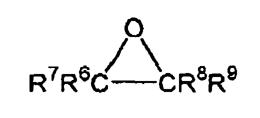 in the presence of a catalyst to form a hydroxy ether;
(b) further reacting the hydroxy ether formed with an aldehyde or a carboxylic acid, acid halide, ester, or anhydride corresponding to the structure of X, defined above, optionally in the presence of a catalyst which does not induce undue polymerisation or rearrangement of the hydroxyether under the reaction conditions; and
(c) recovering the reactive diluent.

13. A process of Claim 12 wherein the carboxylic acids, anhydrides, esters or acyl halides used in the esterification step are selected from the group consisting of oxalic acid, fumaric acid, maleic acid or anhydride, phthalic acid or anhydride and trimellitic acid or anhydride, succinic acid, adipic acid, α- and/or β-hydromuconic acid, malonic acid, nylonate acids, diethyl maleate, fumaryl chloride, and mixtures thereof.

14. A process according to Claim 12 wherein the epoxidation step is carried out with one or more epoxides selected from ethylene oxide, propylene oxide, butadiene mono-oxide, cyclohexene oxide and styrene oxide.

15. A process according to Claim 12 wherein an esterification step is carried out in the presence of an amphoteric catalyst.

16. A process according to any of Claims 9 to 15 wherein the reactive diluent is as defined in any of Claims 1 to 8.

17. An ether ester selected from di-(2-(2,7 octadienoxy)ethyl) dihydromuconate; di-(2-(2,7 octadienoxy)ethyl) fumarate; di-(2-(2,7 octadienoxy)ethyl) succinate; di-(2-(2,7 octadienoxy)ethyl) adipate; di-(2-(2,7 octadienoxy)ethyl) phthalate; di-(2-ethylhexenoxy)ethyl dihydromuconate; 2-(2-ethylhexenoxy)ethyl maleate; 2-(2-ethylhexenoxy)ethyl fumarate; 2-(2-ethylhexenoxy)ethyl succinate; 2-ethyl hexenyl-(2-ethylhexenoxy)ethyl maleate; 2-ethyl hexenyl-(2-ethylhexenoxy)ethyl fumarate; di- (1-(octadienoxy)2-propyl) maleate; tri-(1-(octadienoxy)2-propyl) trimellitate; di-(1-(2-ethylhexenoxy)2-propyl) maleate; tri-(1-(2-ethylhexenoxy)2-propyl) trimellitate; octadienyl-(2-(2,7 octadienoxy) ethyl) maleate; or octadienyl-(2-(2,7 octadienoxy) ethyl) fumarate.

18. A coating formulation comprising a binder resin and a reactive diluent comprising one or more ether esters selected from the group consisting of di-(2-(2,7 octadienoxy)ethyl) dihydromuconate; di-(2-(2,7 octadienoxy)ethyl) fumarate; di-(2-(2,7 octadienoxy)ethyl) maleate; di-(2-(2,7 octadienoxy)ethyl) succinate; di-(2-(2,7 octadienoxy)ethyl) adipate; di-(2-(2,7 octadienoxy)ethyl) phthalate; di-(2-ethylhexenoxy)ethyl dihydromuconate; tri-(2-(2,7 octadienoxy)ethyl) trimellitate; 2-(2-ethylhexenoxy)ethyl maleate; 2-(2-ethylhexenoxy)ethyl fumarate; 2-(2-ethylhexenoxy)ethyl succinate; 2-ethyl hexenyl-(2-ethylhexenoxy)ethyl maleate; 2-ethyl hexenyl-(2-ethylhexenoxy)ethyl fumarate; di- (1-(octadienoxy)2-propyl) maleate; tri-(1-(octadienoxy)2-propyl) trimellitate; di-(1-(2-ethylhexenoxy)2-propyl) maleate; tri-(1-(2-ethylhexenoxy)2-propyl) trimellitate; octadienyl-(2-(2,7 octadienoxy) ethyl) maleate; and octadienyl-(2-(2,7 octadienoxy) ethyl) fumarate.

19. A reactive diluent of formula (I) defined in Claim 1 wherein the diluent has a viscosity below 300 mPa.s and a boiling point above 250°C.

20. A reactive diluent according to Claim 19 and further defined by any of Claims 2 to 8.

21. The use of a reactive diluent of formula (I) as defined in any of Claims 1 to 8 and 18 as a component of a coating formulation.

22. The use of an ether ester composition according to Claim 17 as a component of a coating composition.

## Patentansprüche

1. Beschichtungs-Formulierung, umfassend (i) ein Bindemittel-Harz und (ii) ein reaktives Verdünnungs- bzw. Streckmittel, umfassend eine Allyloxy-Verbindung:
[R*(OCR⁶R⁷-CR⁸R⁹)ₚ-O]ₐ-Z (I)
wobei
R* eine allylische Hydrocarbyl- oder eine allylische Hydrocarbyloxy-hydrocarbyl-Gruppe ist, enthaltend bis zu 22 Kohlenstoff-Atome;
R⁶, R⁷, R⁸ und R⁹
(i) die gleiche oder unterschiedliche Gruppe(n), ausgewählt aus H, einer Alkyl-, einer Alkenyl- und einer Aryl-Gruppe darstellen
oder
(ii) zusammen eine Cyclohexyl-Gruppe darstellen;
Z ausgewählt ist aus: 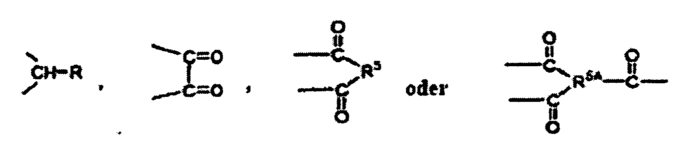 wobei
R⁵ einen divalenten und R^{5A} einen trivalenten gesättigten oder ungesättigten Hydrocarbylen- oder Sauerstoff-enthaltenden Hydrocarbylen-Rest darstellen, abgeleitet aus einem entsprechenden zur Veresterung verwendeten Edukt, umfassend eine Carbonsäure, einen Ester, Säurehalogenid oder Anhydrid, die/der/das 2-20 Kohlenstoff-Atome aufweist,
p einen Wert von 0 bis 5 für jede Allylhydrocarbyloxy- oder Allyletherhydrocarbyloxy-Gruppe unabhängig darstellt und mindestens 1 für mindestens eine solche Gruppe ist und
a 2 oder 3 ist und der Anzahl der in Z vorhandenen Bindungsstellen entspricht.

2. Beschichtungs-Formulierung nach Anspruch 1, wobei das Bindemittel-Harz ein Alkyd-Harz umfasst.

3. Beschichtungs-Formulierung nach Anspruch 1, wobei R* die folgende Struktur aufweist: 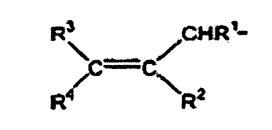 wobei
R¹ H oder eine C₁-C₄-Alkyl-Gruppe oder eine Hydrocarbyloxyalkyl-Gruppe ist, enthaltend bis zu 10 Kohlenstoff-Atome;
R² H oder eine C₁-C₄-Alkyl-Gruppe oder eine Hydrocarbyloxyalkyl-Gruppe ist, enthaltend bis zu 10 Kohlenstoff-Atome;
R³ H oder eine C₁-C₄-Alkyl-Gruppe ist;
R⁴ H, eine Alkyl-Gruppe mit einer geraden oder verzweigten Kette, die bis zu 8 Kohlenstoff-Atome aufweist,
eine Alkenyl-Gruppe, die bis zu 8 Kohlenstoff-Atome aufweist,
eine Aryl-Gruppe oder eine Aralkyl-Gruppe, die bis zu 12 Kohlenstoff-Atome aufweist, oder
eine Hydrocarbyloxyalkyl-Gruppe, die bis zu 10 Kohlenstoff-Atome aufweist, ist oder
R⁴ zusammen mit R¹ eine cyclische Alkylen-Gruppe bildet, unter der Voraussetzung, dass R² H ist; und
in welcher mindestens eine Gruppe, R¹, R², R³ oder R⁴, nicht Wasserstoff ist.

4. Beschichtungs-Formulierung nach Anspruch 1, wobei R* von allylischen Alkoholen mit der folgenden Formel abgeleitet ist: 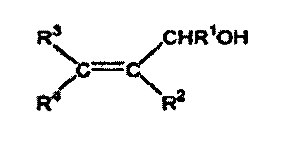 wobei
R¹ H oder eine C₁-C₄-Alkyl-Gruppe oder eine Hydrocarbyloxyalkyl-Gruppe ist, enthaltend bis zu 10 Kohlenstoff-Atome;
R² H oder eine C₁-C₄-Alkyl-Gruppe oder eine Hydrocarbyloxyalkyl-Gruppe ist, enthaltend bis zu 10 Kohlenstoff-Atome;
R³ H oder eine C₁-C₄-Alkyl-Gruppe ist;
R⁴ H, eine Alkyl-Gruppe mit einer geraden oder verzweigten Kette, die bis zu 8 Kohlenstoff-Atome aufweist,
eine Alkenyl-Gruppe, die bis zu 8 Kohlenstoff-Atome aufweist,
eine Aryl-Gruppe oder eine Aralkyl-Gruppe, die bis zu 12 Kohlenstoff-Atome aufweist, oder
eine Hydrocarbyloxyalkyl-Gruppe, die bis zu 10 Kohlenstoff-Atome aufweist, ist oder
R⁴ zusammen mit R¹ eine cyclische Alkylen-Gruppe bildet, unter der Voraussetzung, dass R² H ist; und
in welcher mindestens eine Gruppe, R¹, R², R³ oder R⁴, nicht Wasserstoff ist.

5. Beschichtungs-Formulierung nach Anspruch 3 oder 4, wobei R¹, R² und R³ einzeln ausgewählt sind aus Wasserstoff, Methyl- und Ethyl-Gruppen und R⁴ ausgewählt ist aus Alkenyl-Gruppen, enthaltend bis zu 7 Kohlenstoff-Atome, ArylGruppen oder Aralkylen-Gruppen, die bis zu 10 Kohlenstoff-Atome aufweisen, oder Hydrocarbyloxyalkylen-Gruppen, die bis zu 8 Kohlenstoff-Atome aufweisen.

6. Beschichtungs-Formulierung nach Anspruch 5, wobei R* ausgewählt ist aus 2-Ethylhex-2-en-1-; 2,7-Octadien-; 2-Ethylallyl-; Hept-3-en-2-; 4-Methylpent-3-en-2-; 4-t-Butoxybut-2-en-1-; 4-(α-Methylbenzyloxy)but-2-en-1-; 4-(α-Di-methylbenzyloxy)but-2-en-1-; 4-n-Butoxybut-2-en-1-; 3-Phenyl-2-propen- und 3,5,5-Trimethyl-2-cyclohexen-Gruppen.

7. Beschichtungs-Formulierung nach Anspruch 2 oder 3 , wobei der allylische Alkohol ausgewählt ist aus der Gruppe, bestehend aus 2-Ethylhex-2-en-1-ol; 2,7-Octadienol; 2-Ethylallylalkohol; Hept-3-en-2-ol; 4-Methylpent-3-en-2-ol; 4-t-Butoxybut-2-en-1-ol; 4-(α-Methylbenzyloxy)but-2-en-1-ol; 4-(α-Dimethylbenzyloxy)but-2-en-1-ol; 4-n-Butoxybut-2-en-1-ol; Zimtalkohol und Isophorol.

8. Beschichtungs-Formulierung nach jedem beliebigen vorherigen Anspruch, wobei jeder Hydrocarbylen-Rest R⁵ und R^{5A} eine lineare oder verzweigte, aliphatische, cycloaliphatische oder aromatische, gesättigte oder ungesättigte Alkylen-Gruppe, Alkenylen-Gruppe, Arylen-Gruppe, Arenylen-Gruppe, Alkarylen-Gruppe oder eine Aralkylen-Gruppe ist, enthaltend bis zu 15 Kohlenstoff-Atome.

9. Beschichtungs-Formulierung nach jedem beliebigen der Ansprüche 1 bis 8, umfassend eine oder mehr Komponenten aus Alkyd-Harzen, ungesättigten Polyestern oder Fettsäure-modifizierten Acrylen.

10. Beschichtungs-Formulierung nach Anspruch 9, wobei die relativen Anteile des reaktiven Verdünnungs- bzw. Streckmittels zu Alkyd-Harz im Bereich von 5:95 bis 50:50 Gewichtsteilen liegen.

11. Beschichtungs-Formulierung nach jedem beliebigen der Ansprüche 1 bis 10, enthaltend eine oder mehr weitere Komponente(n), ausgewählt aus der Gruppe, bestehend aus einem Katalysator, einem Trocknungsmittel, einem Antischälungsmittel, Pigmenten, Wasser-Absorbern ("scavengers") und Pigment-Stabilisatoren.

12. Verfahren zur Bildung eines reaktiven Verdünnungs- bzw. Streckmittels mit der in Anspruch 1 definierten Formel (I), umfassend:
(a) Umsetzung eines allylischen Alkohols R*OH mit einem Epoxid 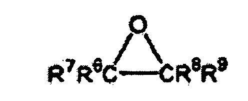 in Gegenwart eines Katalysators zur Bildung eines Hydroxyethers;
(b) weitere Umsetzung des Hydroxyethers, gebildet mit einem Aldehyd oder einer Carbonsäure, einem Säurehalogenid, Ester oder Anhydrid, entsprechend der Struktur von X, wie oben definiert, gegebenenfalls in Gegenwart eines Katalysators, welcher die Polymerisation oder Umlagerung des Hydroxyethers unter den Reaktionsbedingungen nicht maßgeblich induziert; und
(c) Gewinnung des reaktiven Verdünnungs- bzw. Streckmittels.

13. Verfahren nach Anspruch 12, wobei die im Veresterungs-Schritt verwendeten Carbonsäuren, Anhydride, Ester oder Acylhalogenide ausgewählt sind aus der Gruppe, bestehend aus Oxalsäure, Fumarsäure, Maleinsäure oder -Anhydrid, Phthalsäure oder -Anhydrid und Trimellitsäure oder -Anhydrid, Succinsäure, Adipinsäure, α- und/oder β-Hydromukonsäure, Malonsäure, Nylonat-Säuren, Diethylmaleat, Fumarylchlorid, und Mischungen davon.

14. Verfahren gemäß Anspruch 12, wobei der Epoxidierungs-Schritt mit einem oder mehr Epoxid(en) durchgeführt wird, ausgewählt aus Ethylenoxid, Propylenoxid, Butadienmonooxid, Cyclohexenoxid und Styroloxid.

15. Verfahren gemäß Anspruch 12, wobei der Veresterungs-Schritt in Gegenwart eines amphoteren Katalysators durchgeführt wird.

16. Verfahren gemäß jedem beliebigen der Ansprüche 9 bis 15, wobei das reaktive Verdünnungs- bzw. Streckmittel wie in jedem beliebigen der Ansprüche 1 bis 8 definiert ist.

17. Etherester, ausgewählt aus Di-(2-(2,7-octadienoxy)ethyl)dihydromukonat; Di-(2-(2,7-octadienoxy)ethyl)fumarat; Di-(2-(2,7-octadienoxy)ethyl)succinat; Di-(2-(2,7-octadienoxy)ethyl)adipat; Di-(2-(2,7-octadienoxy)ethyl)phthalat; Di-(2-ethylhexenoxy)ethyldihydromukonat; 2-(2-Ethylhexenoxy)ethylmaleat; 2-(2-Ethylhexenoxy)ethylfumarat; 2-(2-Ethylhexenoxy)ethylsuccinat; 2-Ethylhexenyl-(2-ethylhexenoxy)ethylmaleat; 2-Ethylhexenyl-(2-ethylhexenoxy)ethylfumarat; Di-(1-(octadienoxy)2-propyl)maleat; Tri-(1-(octadienoxy)2-propyl)trimellitat; Di-(1-(2-ethylhexenoxy)2-propyl)maleat; Tri-(1-(2-ethylhexenoxy)2-propyl)trimellitat; Octadienyl-(2-(2,7-octadienoxy)ethyl)maleat oder Octadienyl-(2-(2,7-octadienoxy)ethyl)fumarat.

18. Beschichtungs-Formulierung, umfassend ein Bindemittel-Harz und ein reaktives Verdünnungs- bzw. Streckmittel, umfassend einen oder mehr Etherester, ausgewählt aus der Gruppe, bestehend aus Di-(2-(2,7-octadienoxy)ethyl)dihydromukonat; Di-(2-(2,7-octadienoxy)ethyl)fumarat; Di-(2-(2,7-octadienoxy)ethyl)maleat; Di-(2-(2,7-octadienoxy)ethyl)succinat; Di-(2-(2,7-octadienoxy)ethyl)adipat; Di-(2-(2,7-octadienoxy)ethyl)phthalat; Di-(2-ethylhexenoxy)ethyldihydromukonat; Tri-(2-(2,7-octadienoxy)ethyl)trimellitat; 2-(2-Ethylhexenoxy)ethylmaleat; 2-(2-Ethylhexenoxy)ethylfumarat; 2-(2-Ethylhexenoxy)ethylsuccinat; 2-Ethylhexenyl-(2-ethylhexenoxy)ethylmaleat; 2-Ethylhexenyl-(2-ethylhexenoxy)ethylfumarat; Di-(1-(octadienoxy)2-propyl)-maleat; Tri-(1-(octadienoxy)2-propyl)trimellitat; Di-(1-(2-ethylhexenoxy)2-propyl)maleat; Tri-(1-(2-ethylhexenoxy)2-propyl)trimellitat; Octadienyl-(2-(2,7-octadienoxy)ethyl)maleat und Octadienyl-(2-(2,7-octadienoxy)ethyl)fumarat.

19. Reaktives Verdünnungs- bzw. Streckmittel mit der in Anspruch 1 definierten Formel (I), wobei das Verdünnungs- bzw. Streckmittel eine Viskosität unter 300 mPa s und einen Siedepunkt über 250°C aufweist.

20. Reaktives Verdünnungs- bzw. Streckmittel gemäß Anspruch 19 und weiter durch jeden beliebigen der Ansprüche 2 bis 8 definiert.

21. Verwendung eines reaktiven Verdünnungs- bzw. Streckmittels mit der wie in jedem beliebigen der Ansprüche 1 bis 8 und 18 definierten Formel (I) als Komponente einer Beschichtungs-Formulierung.

22. Verwendung einer Etherester-Zusammensetzung gemäß Anspruch 17 als eine Komponente einer Beschichtungs-Zusammensetzung.

## Revendications

1. Formulation de revêtement comprenant (i) un liant et (ii) un diluant réactif comprenant un composé allyloxyle :
[R*(OCR⁶R⁷-CR⁸R⁹)ₚ-O]ₐ-Z (I)
dans laquelle :
R* est un groupement hydrocarbyle allylique ou un groupement hydrocarbyloxyhydrocarbyle allylique contenant jusqu'à 22 atomes de carbone ;
R⁶, R⁷, R⁸ et R⁹ représentent :
(i) le même ou différents groupements choisis parmi H, un groupement alkyle alcényle ou aryle, ou
(ii) pris ensemble un groupement cyclohexyle ;
Z est choisi parmi : 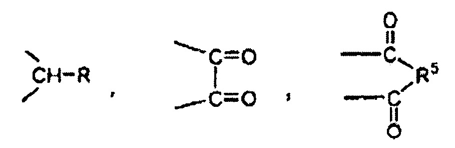 ou 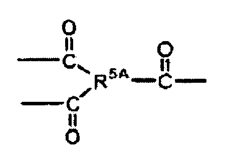 dans lesquelles :
R⁵ représente un groupement divalent et R^{5A} représente un groupement trivalent hydrocarbylène ou hydrocarbylène oxygéné saturé ou insaturé dérivé d'un réactif correspondant utilisé pour l'estérification comprenant un acide carboxylique, un ester, un halogénure d'acyle ou un anhydride, ayant de 2 à 20 atomes de carbone ;
p représente indépendamment une valeur de 0 à 5 pour chaque groupement allylhydrocarbyloxyle ou allyletherhydrocarbyloxyle et est égal à au moins 1 pour au moins l'un de ces groupements, et
a vaut 2 ou 3 et correspond au nombre de sites de liaison présents dans Z

2. Formulation de revêtement selon la revendication 1 dans laquelle la résine liante comprend une résine alkyde.

3. Formulation de revêtement selon la revendication 1 dans laquelle R* a la structure : 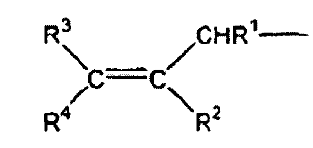 dans laquelle :
R¹ est H ou un groupement alkyle en C₁-C₄ ou un groupement hydrocarbyloxyalkyle contenant jusqu'à 10 atomes de carbone,
R² est H ou un groupement alkyle en C₁-C₄ ou un groupement hydrocarbyloxyalkyle contenant jusqu'à 10 atomes de carbone,
R³ est H ou un groupement alkyle en C₁-C₄,
R⁴ est H, un groupement alkyle linéaire ou ramifié ayant jusqu'à 8 atomes de carbone,
un groupement alcényle ayant jusqu'à 8 atomes de carbone,
un groupement aryle ou un groupement aralkyle ayant jusqu'à 12 atomes de carbone,
ou un groupement hydrocarbyloxyalkyle ayant jusqu'à 10 atomes de carbone, ou
R⁴ pris ensemble avec R¹ forme un groupement alkylène cyclique, à condition que R² soit H ; et
dans laquelle au moins un groupement R¹, R², R³ ou R⁴ n'est pas un hydrogène.

4. Formulation de revêtement selon la revendication 1 dans laquelle R* est dérivé d'alcools allyliques de formule : 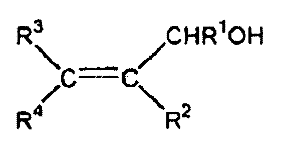 dans laquelle :
R¹ est H ou un groupement alkyle en C₁-C₄ ou un groupement hydrocarbyloxyalkyle contenant jusqu'à 10 atomes de carbone,
R² est H ou un groupement alkyle en C₁-C₄ ou un groupement hydrocarbyloxyalkyle contenant jusqu'à 10 atomes de carbone,
R³ est H ou un groupement alkyle en C₁-C₄,
R⁴ est H, un groupement alkyle linéaire ou ramifié ayant jusqu'à 8 atomes de carbone,
un groupement alcényle ayant jusqu'à 8 atomes de carbone,
un groupement aryle ou un groupement aralkyle ayant jusqu'à 12 atomes de carbone,
ou un groupement hydrocarbyloxyalkyle ayant jusqu'à 10 atomes de carbone, ou
R⁴ pris ensemble avec R¹ forme un groupement alkylène cyclique, à condition que R² soit H ; et
dans laquelle au moins un groupement R¹, R², R³ ou R⁴ n'est pas un hydrogène.

5. Formulation de revêtement selon la revendication 3 ou 4 dans laquelle R¹, R² et R³ sont choisis individuellement parmi des groupements hydrogènes, méthyles et éthyles et R⁴ est choisi parmi des groupements alcènyles contenant jusqu'à 7 atomes de carbone, des groupements aryles ou des groupements aralkylènes ayant jusqu'à 10 atomes de carbone, ou des groupements hydrocarbyloxyalkylènes ayant jusqu'à 8 atomes de carbone.

6. Formulation de revêtement selon la revendication 5 dans laquelle R* est choisi parmi les groupements 2-éthyl-hex-2-èn-1-, 2,7-octadièn-, 2-éthylallyl-, hept-3-èn-2-, 4-méthyl-pent-3-èn-2-, 4-tertiobutoxy-but-2-èn-1-, 4-(α-méthylbenzyloxy)-but-2-èn-1-, 4-(α-diméthylbenzyloxy)-but-2-èn-1-, 4-n-butoxy-but-2-èn-1-, 3-phényl-2-propèn- et 3,5,5-triméthyl-2-cyclohexèn-.

7. Formulation de revêtement selon la revendication 2 ou 3 dans laquelle l'alcool allylique est choisi dans le groupe constitué du 2-éthyl-hex-2-èn-1-ol, du 2,7-octadiénol, de l'alcool 2-éthylallylique, du hept-3-èn-2-ol, du 4-méthyl-pent-3-èn-2-ol, du 4-tertiobutoxy-but-2-èn-1-ol, du 4-(α-méthylbenzyloxy)-but-2-èn-1-ol, du 4-(α-diméthylbenzyloxy)-but-2-èn-1-ol, du 4-n-butoxy-but-2-èn-1-ol, de l'alcool cynnamylique et de l'isophorol.

8. Formulation de revêtement selon l'une quelconque des revendications précédentes dans laquelle chaque groupement hydrocarbylène R⁵ et R^{5A} est un groupement alkylène, alcénylène, arylène, arénylène, alkarylène, ou aralkylène, linéaire ou ramifié, aliphatique, cycloaliphatique ou aromatique, saturé ou insaturé, contenant jusqu'à 15 atomes de carbone.

9. Formulation de revêtement selon l'une quelconque des revendications 1 à 8 comprenant une ou plusieurs résines alkydes, polyesters insaturés ou acryliques modifiés par acides gras.

10. Formulation de revêtement selon la revendication 9 dans laquelle les proportions relatives du diluant réactif par rapport à la résine alkyde se situe dans la gamme de 5:95 à 50:50 parts en poids.

11. Formulation de revêtement selon l'une quelconque des revendications 1 à 10 contenant un ou plusieurs autres composants choisis dans le groupe consistant en un catalyseur, un siccatif, un agent antipeaux, des pigments, des éliminateurs d'eau et des stabilisateurs de pigments.

12. Procédé pour former un diluant réactif selon la formule (I) définie à la revendication 1, comprenant :
(a) la réaction d'un alcool allylique R*OH avec un époxyde : 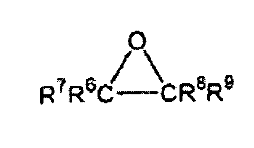 en présence d'un catalyseur pour former un hydroxyéther,
(b) suivie de la réaction de l'hydroxyéther formé avec un aldéhyde ou un acide carboxylique, halogénure d'acyle, ester ou anhydride correspondant à la structure de X, défini plus haut, facultativement en présence d'un catalyseur qui n'induise pas de polymérisation ni de réarrangement indu de l'hydroxyéther dans les conditions de la réaction, et
(c) la récupération du diluant réactif.

13. Procédé selon la revendication 12 dans lequel les acides carboxyliques, anhydrides, esters ou halogénures d'acyle utilisés dans l'étape d'estérification sont choisis dans le groupe constitué de l'acide oxalique, l'acide fumarique, l'acide ou l'anhydride maléique, l'acide ou l'anhydride phtalique, et l'acide ou l'anhydride trimellitique, l'acide succinique, l'acide adipique, les acides α- et/ou β-hydromuconiques, l'acide malonique, les acides nylonates, le maléate de diéthyle, le chlorure de fumaryle, et leurs mélanges.

14. Procédé selon la revendication 12 dans lequel l'étape d'époxydation est réalisée avec un ou plusieurs époxydes choisis parmi l'oxyde d'éthylène, l'oxyde de propylène, le monoxyde de butadiène, l'oxyde de cyclohexène et l'oxyde de styrène.

15. Procédé selon la revendication 12 dans lequel l'étape d'époxydation est réalisée en présence d'un catalyseur amphotère.

16. Procédé selon l'une quelconque des revendications 9 à 15 dans lequel le diluant réactif est comme défini dans l'une quelconque des revendications 1 à 8.

17. Ether-ester choisi parmi le dihydromuconate de di-2-(2,7-octadiénoxy)-éthyle, le fumarate de di-2-(2,7-octadiénoxy)-éthyle, le succinate de di-2-(2,7-octadiénoxy)-éthyle, l'adipate de di-(2-(2,7-octadiénoxy)-éthyle), le phtalate de di-(2-(2,7-octadiénoxy)-éthyle), le dihydromuconate de di-(2-éthylhexénoxy)-éthyle), le maléate de 2-(2-éthylhexénoxy)-éthyle, le fumarate de 2-(2-éthylhexénoxy)-éthyle, le succinate de 2-(2-éthylhexénoxy)-éthyle, le maléate de 2-éthylhexényl-(2-éthylhexénoxy)-éthyle, le fumarate de 2-éthylhexényl-(2-éthylhexénoxy)-éthyle, le maléate de di-(1-(octadiénoxy)-2-propyle), le trimellitate de tri-(1-(octadiénoxy)-2-propyle), le maléate de di-(1-(2-éthylhexénoxy)-2-propyle), le trimellitate de tri-(1-(2-éthylhexénoxy)-2-propyle), le maléate d'octadiényl-(2-(2,7-octadiénoxy)-éthyle) ou le fumarate d'octadiényl-(2-(2,7-octadiénoxy)-éthyle).

18. Formulation de revêtement comprenant une résine liante et un diluant réactif comprenant un ou plusieurs éther-esters choisis dans le groupe constitué du dihydromuconate de di-(2-(2,7-octadiénoxy)-éthyle), du fumarate de di-(2-(2,7-octadiénoxy)-éthyle), du maléate de di-(2-(2,7-octadiénoxy)-éthyle), du succinate de di-(2-(2,7-octadiénoxy)-éthyle), de l'adipate de di-(2-(2,7-octadiénoxy)-éthyle), du phtalate de di-(2-(2,7-octadiénoxy)-éthyle), du dihydromuconate de di-(2-éthylhexénoxy)-éthyle), du trimellitate de tri-(2-(2,7-octadiénoxy)-éthyle), du maléate de 2-(2-éthylhexénoxy)-éthyle), du fumarate de 2-(2-éthylhexénoxy)-éthyle), du succinate de 2-(2-éthylhexénoxy)-éthyle), du maléate de 2-éthyl-hexényl-(2-éthylhexénoxy)-éthyle, du fumarate de 2-éthyl-hexényl-(2-éthylhexénoxy)-éthyle, du maléate de di-(1-octadiénoxy)-2-propyle, du trimellitate de tri-(1-octadiénoxy)-2-propyle, du maléate de di-(1-(2-éthylhexénoxy)-2-propyle), du trimellitate de tri-(1-(2-éthylhexénoxy)-2-propyle), du maléate d'octadiényl-(2-(2,7-octadiénoxy)-éthyle et du fumarate d'octadiényl-(2-(2,7-octadiénoxy)-éthyle.

19. Diluant réactif selon la formule (I) définie à la revendication 1 dans lequel le diluant a une viscosité inférieure à 300 mPa.s et un point d'ébullition supérieur à 250°C.

20. Diluant réactif selon la revendication 19 défini en outre par l'une quelconque des revendications 2 à 8.

21. Utilisation d'un diluant réactif selon la formule (I) comme défini dans l'une quelconque des revendications 1 à 8 et 18 comme composant d'une formulation de revêtement.

22. Utilisation d'une composition éther-ester selon la revendication 17 comme composant d'une formulation de revêtement.
